(19)

Europäisches
Patentamt
European
Patent Office
Office européen
des brevets

(11)  **EP 4 306 051 A1**

(12)  # EUROPEAN PATENT APPLICATION

(43) Date of publication:
**17.01.2024  Bulletin 2024/03**

(21) Application number: **22184948.2**

(22) Date of filing: **14.07.2022**

(51) International Patent Classification (IPC):
**A61B 5/287** (2021.01)  **A61B 5/367** (2021.01)
**A61B 5/06** (2006.01)  **A61B 34/20** (2016.01)

(52) Cooperative Patent Classification (CPC):
**A61B 5/287; A61B 5/061; A61B 5/367;**
A61B 2034/105; A61B 2034/2051; A61B 2034/256;
A61B 2090/3954; A61B 2560/0238

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **TORBJØRN, Vik**
  **Eindhoven (NL)**

• **SCHÄFER, Dirk**
  **Eindhoven (NL)**
• **WILDEBOER, Rogier Rudolf**
  **5656AG Eindhoven (NL)**
• **BUDZELAAR, Franciscus Paulus Maria**
  **5656AG Eindhoven (NL)**

(74) Representative: **Philips Intellectual Property &
Standards
High Tech Campus 52
5656 AG Eindhoven (NL)**

(54)  **DETERMINING A DISTANCE BETWEEN PAIRS OF FIELD-SENSITIVE SENSORS OF AN INTERVENTIONAL DEVICE**

(57)  A mechanism for determining an inter-sensor distance between at least one pair of field-sensitive sensors of an interventional device. A minimization process is used to determine the inter-sensor distance, which minimizes and/or converges a difference between an estimated distance or estimated distances and a function-derived distance or function-derived distances by modifying the estimated distance(s). The function-derived distances are distances determining using a measurement-to-position mapping function that maps sets of measurements of field-sensitive sensors to positions in a spatial coordinate system

FIG. 3

EP 4 306 051 A1

**Description**

FIELD OF THE INVENTION

The present invention relates to the field of interventional devices, and in particular to interventional devices carrying a plurality of field-sensitive sensors.

BACKGROUND OF THE INVENTION

Intrabody interventional devices are often used to investigate and treat anatomical cavities and anatomical elements within a subject or patient. For performing effective treatment, it is important for a clinician to be able to track the location of an interventional device.

One approach for tracking an interventional device is to provide the interventional device with two or more sensors, which can be labelled field-sensitive sensors. The measurements recorded at the two or more field-sensitive sensors caused by crossing electromagnetic fields induced in the subject change as the position of the interventional device changes. Examples of suitable measurements include a voltage (measurement) or a magnetic response (i.e., a measurement responsive to a change in a magnetic field). The measurement changes can be used to track the location of the interventional device. One system describing such type of tracking for a catheter having multiple sensors is disclosed in the EP 3568068 A1.

By taking advantage of known sensor distances on the interventional device, a processing system can determine a function that maps from the set of measurements (e.g., a set of measurements recorded at a single sensor) to a set of spatial coordinates. This function can be labelled a measurement-to-position ("M2R") function. If the measurement is a voltage or voltage response, this function can be labelled a voltage-to-position ("V2R") function. Typically, determination of the measurement-to-position function is performed by minimizing a mathematical cost function that parametrizes the measurement-to-position function and penalizes deviations between the known inter-sensor distances and the estimated inter-sensor distances from the measurement-to-position function. This mapping can thereby take account of any field distortions within the body that results from different conductivities in different tissue types.

Thus, the inter-sensor distances (i.e., distances between pairs of field-sensitive sensors) are required to tune or accurately determine the measurement-to-position function.

SUMMARY OF THE INVENTION

The inter-sensor distances can vary between interventional devices of different vendors, or, as the inventors have found, between different production batches or even the same production batch of one type of interventional device due to e.g., manufacturing tolerances.

There is thus a need for a method to account for these differences to thereby improve the tracking.

The invention is defined by the claims.

According to examples in accordance with an aspect of the disclosure, there is provided a computer-implemented method of determining, for each of one or more pairs of field-sensitive sensors carried by an interventional device, a distance between said pair of field-sensitive sensors.

The computer-implemented method comprises defining an estimated distance between each pair of field-sensitive sensors. The computer-implemented method also comprises, for each of a plurality of different positions of the interventional device within crossing electromagnetic fields: receiving, a set of measurements for each field-sensitive sensor, each measurement in the set of measurements being a measurement of the field-sensitive sensor to a different one of the crossing electromagnetic fields; predicting a position of each field-sensitive sensor in a spatial co-ordinate system by processing the set of measurements using a measurement-to-position function; predicting, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors. The computer-implemented method also comprises iteratively performing a minimization process until one or more predetermined criteria are met, the minimization process comprising: determining a value of a combined cost function that processes the function derived distances and the estimated distance between each pair of field-sensitive sensors; and modifying the estimated distance between each pair of field-sensitive sensors responsive to the determined value of the combined cost function; after iteratively performing the minimization process, for each of the one or more pairs of field-sensitive sensors, defining the estimated distance between said pair of field-sensitive sensors, as the distance between the pair of field-sensitive sensors.

Approaches recognize that it is possible to calibrate or estimate the true distance between each pair of field-sensitive sensors using a minimization function. The proposed technique provides a mechanism for using a preliminary or initial measurement-to-position function to establish the distance between each pair of field-sensitive sensors, labelled a function-derived distance, for a plurality of positions of the interventional device within crossing electromagnetic fields. The value of a combined cost function is determined, which effectively identifies an average similarity between the function-derived distances and the estimated distance. The value of this cost function is then used as a value for modifying the estimated distance.

Approaches thereby provide a mechanism for automated determination of inter-sensor distances. Embodiments are based on the realization that with sufficient sample size (e.g., with sufficient numbers of positions of the interventional device), the inherent inhomogeneity of the electromagnetic fields is captured or otherwise taken into account in the calculation, and an inter-sensor distance can be identified with a high degree of accuracy.

In some examples, no modifications are made to the measurement-to-position function during the iterative performances of the minimization process. This approach is less computationally expensive, likely more robust and can integrated more closely with existing code/approaches than approaches that modify both the measurement-to-position function and the estimated distance(s).

In some examples, the minimization process further comprises: modifying the measurement-to-position function responsive to the determined value of the combined cost function; repeating the step of predicting a position of each field-sensitive sensor in a spatial co-ordinate system by processing the set of measurements using a measurement-to-position function; and repeating the step of predicting, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors.

This approach allows for simultaneous determination of the measurement-to-position function and the inter-sensor distances, and provides a faster approach for generating the measurement-to-position function.

In some examples, the step of determining the value of the combined cost function comprises: determining, for each of the plurality of different positions of the interventional device, the value of a cost function for each sensor pair using a difference between the function-derived distance and the estimated distance; and determining a value of a combined cost function by combining the determined values of the cost function for each sensor pair. This approach provides a mechanism by which each function-derived distance contributes to the combined cost function, improving the relevance of the cost function and the accuracy of the inter-sensor distance determination.

In some examples, combining the determined values of the cost function comprise summing the determined values of the cost function.

In some embodiments, determining a value of a cost function for each sensor pair comprises, for each sensor pair, determining the square of the difference between the function derived distance and the estimated distance. This approach more heavily penalizes large errors or discrepancies to more quickly and accurately converge the estimated distance(s) to a correct or accurate value.

The one or more pairs of field-sensitive sensors may comprises only a subset of all possible pairs of field-sensitive sensors of the interventional device, the subset comprising a non-integer fraction of the possible pairs of the field-sensitive sensors. In particular, the one or more pairs of field-sensitive sensors may include only those pairs that are used in determining the measurement-to-position function for the interventional device, which can vary depending upon the type or model of the interventional device. This approach increases the speed of determining the inter-sensor distances, as only the most relevant inter-sensor distances are used in the calculations and/or minimization function.

The step of calculating a distance between the positions, in the spatial co-ordinate system, of the field-sensitive sensors in the pair of field-sensitive sensors may comprise calculating the L2 norm of a vector that originates at the position, in the spatial co-ordinate system, of one of the field-sensitive sensors of the pair of field-sensitive sensors and terminates at the position, in the spatial co-ordinate system of the other of the field-sensitive sensors of the pair of field-sensitive sensors. Other suitable approaches for determining a distance will be apparent to the skilled person, e.g., any vector norm or the L1 norm. Use of the L2 norm is particularly advantageous in improving the accuracy of the function-derived distance.

Preferably, the plurality of different positions of the interventional device comprises no fewer than 100 different positions, and more preferably, no fewer than 500 different positions. The greater the number of different positions, the more accurate the determination of the distance(s) between the pair(s) of field-sensitive sensors.

It is appreciated that the greater the number of positions, the more time consuming or resource-intensive the inter-sensor distance determination process will be. Therefore, a representative selection of positions is desired. Thus, in some examples, the plurality of different positions comprises no more than 10,000 different positions, for instance, no more than 5,000 different positions, e.g., no more than 2,000 different positions.

In some examples, the step of defining an estimated distance between each pair of field-sensitive sensors comprises obtaining an estimated distance between each pair of field-sensitive sensors from a user input at a user interface or data stored by a memory unit.

The one or more predetermined criteria may comprise: the minimization process being iteratively performed no less than a first predetermined number of iterations; the value of the combined cost function changing by less than a first predetermined percentage or amount over a preceding second predetermined number of iterations; and/or an override indicator being provided.

In some examples, the computer-implemented method further comprises obtaining, for each pair of field-sensitive sensors, an expected distance between said pair of field-sensitive sensors; determining, for all of the one or more pairs of field-sensitive sensors a measure of similarity between the expected distance and the defined distance; and generating an alert responsive to the measure of similarity exceeding a predetermined threshold.

Each of the plurality of different positions of the interventional device may be a different position within an anatomical cavity of a subject.

There is also proposed a computer program product comprising computer program code means which, when executed on a computing device having a processing system, cause the processing system to perform all of the steps of any herein described method.

There is also proposed a processing system for determining, for each of one or more pairs of field-sensitive sensors carried by an intervention device, a distance between said pair of field-sensitive sensors.

The processing system comprises an input interface configured to receive, for each of a plurality of different positions of the interventional device within crossing electromagnetic fields, a set of measurements for each field-sensitive sensor, each measurement in the set of measurements being a measurement of the field-sensitive sensor to a different one of the crossing electromagnetic fields.

The processing system also comprises a processing unit configured to: define an estimated distance between each pair of field-sensitive sensors; for each of a plurality of different positions of the interventional device within crossing electromagnetic fields: predict a position of each field-sensitive sensor in a spatial co-ordinate system by processing the set of measurements using a measurement-to-position function; predict, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors; iteratively perform a minimization process until one or more predetermined criteria are met, the minimization process comprising: determining a value of a combined cost function that processes the function derived distances and the estimated distance between each pair of field-sensitive sensors; and modifying the estimated distance between each pair of field-sensitive sensors responsive to the determined value of the combined cost function; and after iteratively performing the minimization process, for each of the one or more pairs of field-sensitive sensors, define the estimated distance between said pair of field-sensitive sensors, as the distance between the pair of field-sensitive sensors.

The processing system may further comprise an output interface configured to output, for each of the one or more pairs of field-sensitive sensors, the defined distance.

The skilled person would be readily capable of modifying any herein described processing system to carry out any herein described computer-implemented method, and vice versa. Any advantages of the computer-implemented method according to the present invention analogously and similarly apply to the system and computer program herein disclosed. It will be appreciated by those skilled in the art that two or more of the above-mentioned options, implementations, and/or aspects of the invention may be combined in any way deemed useful.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system for analyzing or investigating the anatomical cavity of a subject;
Fig. 2 illustrates a process for reconstructing an anatomical model;
Fig. 3 illustrates a computer-implemented method according to an embodiment;
Fig. 4 illustrates a minimization process for use in an embodiment;
Fig. 5 illustrates further optional features of embodiments;
Fig. 6 illustrates a processing system according to an embodiment; and
Fig. 7 illustrates a processing system.

DETAILED DESCRIPTION OF THE EMBODIMENTS

The invention will be described with reference to the figures.

The detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The figures are merely schematic and are not drawn to scale. The same reference numerals are used throughout the figures to indicate the same or similar parts.

The invention provides a mechanism for determining an inter-sensor distance between at least one pair of field-sensitive sensors of an interventional device. A minimization process is used to determine the inter-sensor distance, which minimizes and/or converges a difference between an estimated distance or estimated distances and a function-derived distance or function-derived distances by modifying the estimated distance(s). The function-derived distances are distances determining using a mapping function that maps a set of measurements of interventional field-sensitive sensors induced by crossing electromagnetic fields to a position in a spatial co-ordinate system.

In the context of the present disclosure, a distance between a pair of field-sensitive sensors may be an effective distance, i.e., a distance that can be used to generate a mapping function that accounts for field distortions within a desired accuracy level. The distance may, for instance, be a distance between the effective centers of sensitivity of the field-sensitive sensors, e.g., the electrical centers.

For improved contextual understanding of how an electromagnetic field based tracking system operates, the principle and purpose of generating a mapping function for mapping corresponding responses of field-sensitive sensors to a position will be described first. A detailed disclosure may be found in for example EP 3568068 A1, which is incorporated herein by reference in its entirety.

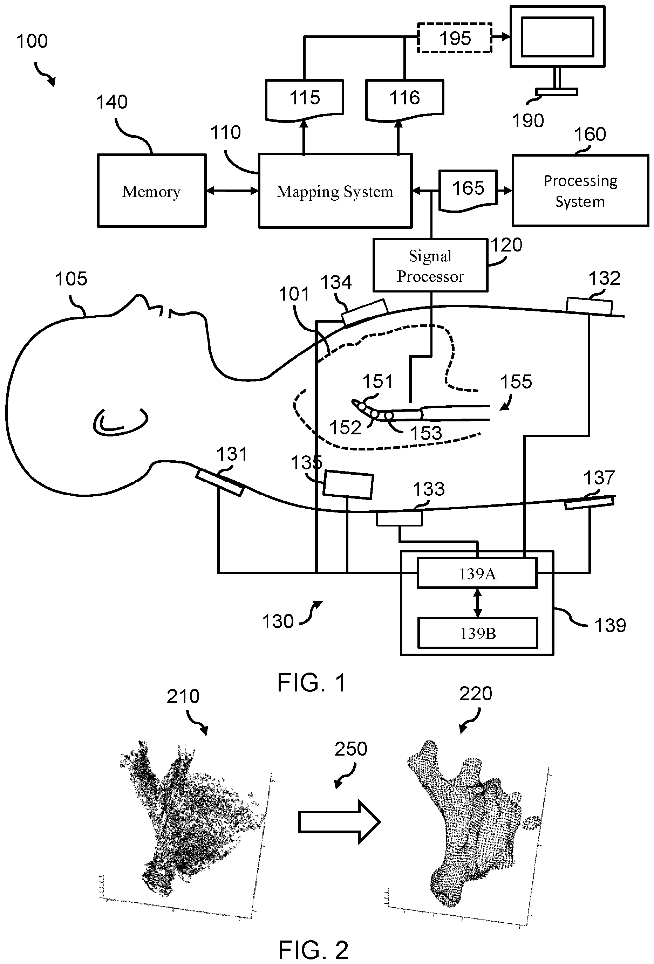

Fig. 1 illustrates a system 100 for analyzing or investigating an anatomical cavity 101 of a subject 105. The anatomical cavity may, for instance, be an anatomical cavity within any suitable anatomical element of the subject, such as the heart, liver, kidney, lungs etc. or parts thereof. Preferably such cavity is an atrium or ventricle of the heart. Examples of such systems are electro-anatomical mapping systems.

An interventional device 150 carries two or more field-sensitive sensors 151, 152, 153. A measurement at a field-sensitive sensor will differ dependent upon the position of the field-sensitive sensor within a particular electromagnetic field. The interventional device may be or comprise a catheter, such as for example an electrophysiological mapping catheter or an ablation catheter, although other devices or catheters may be used.

Raw signals at the two or more field-sensitive sensors 151, 152, 153 may be filtered, sampled and/or digitized using a signal processor 120. In particular, the signal processor may (electrically) connect to the field-sensitive sensors 151, 152, 153 and process the raw signals at the field-sensitive sensors to produce data or sets of measurements for digital processing by a processing system or other similar device. The signal processor 120 may therefore comprise appropriate circuitry for performing such functions, e.g., filtering circuitry, sampling circuitry and/or digitizing circuitry (such as an analogue-to-digital converter).

One approach for monitoring the position of a field-sensitive sensor as the interventional device moves within the anatomical cavity is to monitor a set of measurements for the field-sensitive sensor to crossing electromagnetic fields induced within the subject. Each measurement in the set is a measurement of the field-sensitive sensor to a different one of the crossing electromagnetic fields. Thus, if there are three crossing electromagnetic fields, then each set of measurements will comprise three different measurements.

The signal processor 120 may be appropriately configured for producing the sets of measurements, e.g., to sample and/or filter the raw sensor signals produced by the field-sensitive sensors for digital processing.

One example of a suitable measurement, for an electric field, is a voltage at a sensor. Thus, in some examples, the field-sensitive sensor is an electrode ("device electrode") at which a voltage, for each electromagnetic field, differs depending upon the position of said sensor with respect to the electromagnetic field. In this scenario, the signal processor 120 may be able to sample and/or filter a voltage at each device electrode to produce the set of measurements.

Another example of a suitable measurement, for an electromagnetic field, is a magnetic response. Thus, in some examples, the field-sensitive sensor is a magnetic sensor such as a Hall-effect sensor or a set of coils, at which a measurement captured by the magnetic sensor, for each electromagnetic field, differs depending upon the position of said sensor with respect to the electromagnetic field. In this scenario, the signal processor 120 may be able to sample and/or filter a voltage (or other electrical response) produced by each magnetic sensor to produce the set of measurements.

An electromagnetic field generating arrangement 130 comprises a set of field generators 131, 132, 133, 134, 135, 137 operably connected to a field controller 139. The arrangement may be configured to generate the crossing electromagnetic fields within at least a part of subject 105 using the set of field generators positioned externally with respect to the subject 105. Suitable examples of field generators include electrodes (for generating electric fields) or solenoids or other electromagnetics (for generating magnetic fields). Other suitable examples will be apparent to the skilled person. The field controller 139 is configured to control characteristics (e.g., measurement and/or current) of the electric signals provided to each field generator to thereby generate the crossing fields. For example, the arrangement may generate alternating electromagnetic fields as the crossing electromagnetic fields.

The field controller 139 may comprise a suitable signal generator 139A for generating and transmitting signals to each field generator. The operation of the signal generator 139A may be controlled by a field controller processing system 139B of the field controller 139 (e.g., which may form part of any other processing circuitry of the system 100).

To distinguish between different measurements, each electromagnetic field may be generated with a different and distinguishable frequency and/or temporal code. Thus a "measurement" may be an average or instantaneous measurement at a particular frequency and/or temporal code, corresponding to the frequency and/or temporal code of the corresponding electromagnetic field.

The field controller 139 may be configured to control the electromagnetic fields to operate in the frequency range of 10 kHz to 1 MHz, preferably in the range of 10-100 kHz. This frequency range is particularly useful for ensuring penetration of a subject, whilst providing a frequency range that attenuates in human tissue without significant damage/injury to the tissue.

The reference sensor (e.g., 137) can serve, if required for the type of measurement, as a reference, e.g., a measurement reference, for all measurements taken by sensors, e.g., as a reference for the response of the device sensors.

The set of the measurements of a field-sensitive sensor 151, 152, 153 (to the electromagnetic fields) changes as the relative position of the field-sensitive sensor moves about the subject. Such movement may be caused by a physician deliberately roving or moving the field-sensitive sensors within a cavity 101 of the subject 105, to collect measurements at different location within the subject 105 (or the cavity 101). The principle of crossing electromagnetic fields thereby facilitates tracking of the relative position of the interventional device using a mapping system 110 that monitors the measurements of any field-sensitive sensors to the crossing electromagnetic fields (e.g., receives sets of measurements produced by the signal processor). The measurements are obtained or contained within a measurement coordinate system sometimes referred to as a "measurement space".

Due to the inhomogeneity of the electromagnetic field(s) in the subject, it is usually necessary to map the set of measurements of a sensor to a relative position in a subject using a suitable measurement-to-position or mapping function, sometimes referred to as an "M2R function". If the measurement is a voltage or voltage response, this function can be labelled a voltage-to-position ("V2R") function. The mapping function therefore converts a set of measurements in a measurement coordinate system into a location or position (e.g., set of co-ordinates) in a spatial co-ordinate system. This mapping function will determine the relative position(s) of the sensors, and therefore of the interventional device, in a spatial co-ordinate system (e.g., compared to a measurement co-ordinate system). This spatial coordinate system is sometimes referred to as an "R-space". A spatial co-ordinate system is one that represents a true spatial relationship (e.g., proportionally scaled to geometric or physical distances) between elements modelled in the spatial co-ordinate system.

Approaches for generating such a mapping function are well established in the art. Example processes are disclosed by the European Patent Applications EP 0775466 A2, EP 3568068 A1 and EP 3607879 A1.

Generally, generating a mapping function makes use of known inter-sensor distances to calibrate for the inhomogeneity in the electromagnetic fields. Thus, it is typically necessary to know the distance between each of one or more pairs of field-sensitive sensors. Historically, these have been provided by the manufacturer or operator of the interventional device.

Once a number of locations of the interventional device and/or one or more sensors on the interventional device in the spatial co-ordinate system have been established, it is possible to construct an anatomical model 115 of the anatomical cavity 101 (i.e., a cavity in which the interventional device is able to move). This process may also be performed by the system 100 or a subsystem thereof such as for example mapping system 110.

A brief description of how to generate an anatomical model is provided for improved contextual understanding. This description also aids to emphasize a particular use-case scenario for data derived from a measurement-to-position function, and therefore the clinical benefit(s) and advantage(s) of a more accurate measurement-to-position function.

Broadly, the mapping system 110 may build an anatomical model 115 of an anatomical cavity 101 (e.g., a chamber, vessel or void) within a subject by processing a cloud of points. Each point represents a location or position of the interventional device or sensor in the spatial co-ordinate system, and could therefore be represented as a set of data, e.g., a set of co-ordinates.

More specifically, a reconstruction algorithm is used to generate an anatomical model of the (investigated part of the) anatomical cavity. The anatomical model may, for example, be a 3D mesh or surface that depicts or models the (known bounds of the) anatomical cavity. In the present disclosure, the anatomical model comprises a 3D mesh defined by a plurality of vertices, each vertex being associated with a different location in the predefined co-ordinate system. The 3D mesh is therefore defined in the same co-ordinate system as the determined locations of the field-sensitive sensor(s). The 3D mesh may, for instance, be defined as sets of co-ordinates defining vertices of the 3D mesh.

The process of reconstructing an anatomical model from a point cloud is conceptually illustrated in Fig. 2, which demonstrates a process 250 in which a cloud of points 210 ("point cloud") is transformed into an anatomical model 220. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

Referring to Fig. 1, the anatomical model may be stored, for instance, in a memory 140 or storage unit. Of course, the anatomical model may be output for further processing and/or to a user interface 190, such as a screen or display, configured to provide a visual representation of the anatomical model.

Thus, the system 100 may comprise a user interface 190 configured to provide a visual representation of the anatomical model. In some examples, a display processor 195 may be configured to receive the anatomical model 115 from the mapping system 110 and render the anatomical model to generate display data that can be received and processed by the user interface 190 for providing a visual representation of the anatomical model.

More particularly, once a 3D mesh has been generated then a visual representation of the 3D mesh can be provided. Providing a visual representation of the 3D mesh may comprise, for example, rendering a reconstruction of the surface of the (known) anatomical cavity. The visual representation may be provided to a user with an appropriate output device such as a display device. The system 100 may include the appropriate output terminals connectable to or connected to the output device according to known principles.

In one method, the steps of gathering sets of measurements of field-sensitive sensors, generating the mapping function, transforming a set of measurements to a set of positions in R space using a mapping function and reconstructing an anatomical model can be repeated until an acceptable anatomical model has been obtained.

Once an anatomical model has been generated, it is possible to monitor the location of the field-sensitive sensor(s) and/or interventional device with respect to the anatomical model. For example, both the model and the tracking of location of the device may have the shared spatial coordinate system. The system 100 or the mapping system 110 may be accordingly configured to determine a location of the field-sensitive sensor(s) and/or the interventional device. This may comprise identifying or determining one or more (possibly new) locations 116 of each field-sensitive sensor and/or interventional device. This may include sampling additional sets of measurements of the field-sensitive sensors over time and mapping such additional sets to respective additional locations in R-space using the mapping function. A model of the interventional device comprising the known locations of the field-sensitive sensors on the device may be used to generate a location of at least a part of (e.g. preferably the part comprising one or more of the sensors) the interventional device.

The anatomical model in combination with monitored additional (new) locations may be used, for instance, to provide a visual representation of the known bounds of the anatomical cavity (represented by the anatomical model) and the relative position of the interventional device and/or a set of its sensors with respect to the anatomical cavity. This provides useful clinical information to an operator of the interventional device, e.g., to track the progress (e.g. location and/or orientation) of the interventional device and/or view the shape/structure/size of the anatomical cavity in which the interventional device is to be moved or is moving.

As previously explained, to generate an accurate mapping function for converting a set of measurements to a location in a spatial co-ordinate system, it is usually necessary to use known inter-sensor distances to calibrate for the inhomogeneity of the electromagnetic fields.

It has been recognized that, due to imperfections and natural variation in the manufacturing process, it is rare for inter-sensor distances provided by the manufacturer to exactly represent the true inter-sensor distances. For highly accurate mapping or location tracking within an anatomical cavity, there is therefore a need to calibrate the interventional device in that its effective inter-sensor distances are known.

The present disclosure proposes an approach that facilitates calibration or determination of inter-sensor distances for an interventional device "on-the-go", e.g., within the patient or subject, thereby avoiding or obviating the need for an explicit, pre-procedural calibration.

The proposed calibration approach resolves the inter-sensor distances by treating the determination of the inter-sensor distance(s) as a mathematical minimization problem. In one example, this problem can be solved in a separate function or procedure prior to performing a function/procedure for determining or deriving the mapping function. In another example, the two functions/procedures are combined, so that determination of inter-sensor distances is performed together with the determination of the mapping function. A combination of both approaches can also be used.

In any case, the determination of inter-sensor distances can be effectively 'invisible' to the user and so the workflow is simplified. Thus, in one example the sensor distances are not explicitly output by the system such that they are provided (e.g., shown) to a user. In other examples, an output may be generated by the system which output comprises one or more determined sensor distances. Advantageously and optionally the system may provide an output comprising one or more of the determined distances together with corresponding one or more reference distances. Such reference distances may be taken from a memory of the system and they may have been entered to the system by the user manually or by appropriate interventional configuration data. For example, such distances can be part of a pre-calibration data and/or pre-specification data. Such data may for example originate from the system manufacturer or vendor and/or interventional device manufacturer or vendor.

Methods described herein thus include approaches for determining for each of one or more pairs of field-sensitive sensors, a distance between said pair of field-sensitive sensors, wherein each field-sensitive sensor is carried by an interventional device. This mechanism or approach may be employed or carried out by a processing system 160, which produces the estimated difference(s) 165, e.g., for processing by the mapping system 110.

There is therefore proposed a processing system 160, as well as a system 100 comprising the processing system 160.

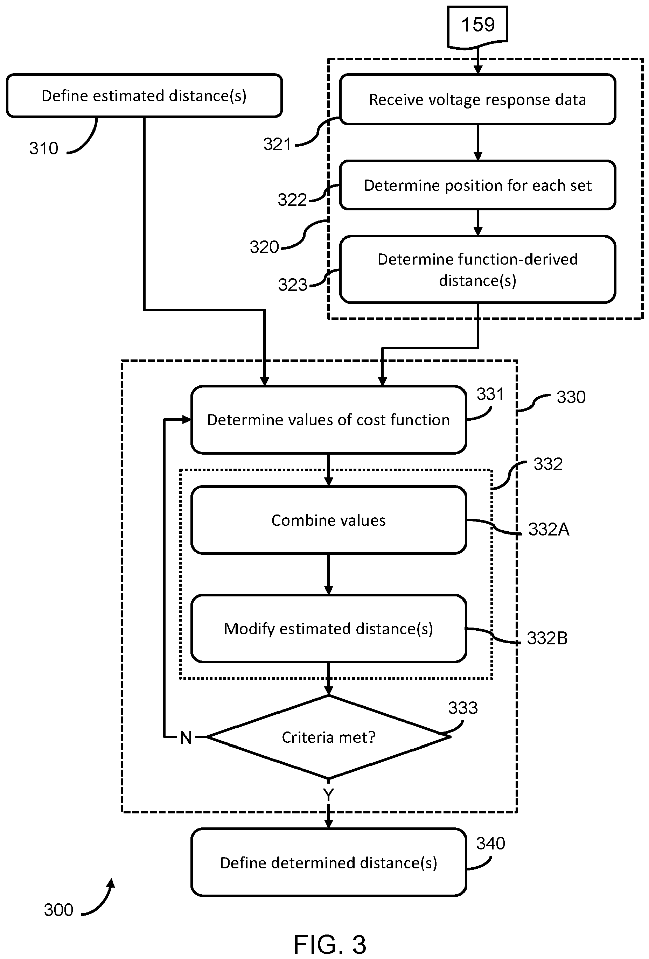

Fig. 3 is a flowchart illustrating a computer-implemented method 300 for determining, for each of one or more pairs of field-sensitive sensors, a distance between said pair of field-sensitive sensors.

Each field-sensitive sensor is carried by an interventional device, such as that previously described (e.g., a catheter). The interventional devices carries one or more different pairs of field-sensitive sensors, e.g., no fewer than three pairs of field-sensitive sensors (i.e., at least three field-sensitive sensors). Note that pairs of sensors do not necessarily have to be defined by neighboring sensors or sensors nearest to each other. Any two sensors on a catheter will define a distance between them and this distance may be determined.

The method 300 may be a passive method that is carried out whilst the interventional device is within the anatomical cavity of the subject. None of the steps performed by the method require any direct physical interaction between the device and the subject, but rather relate to approaches for the mere processing of data.

With reference to Fig. 1, the method 300 may be carried out or executed by the processing system 160. Although illustrated as a separate component, in some examples, the processing system 160 may, for instance, form an aspect or part of the mapping system 110 that generates the measurement-to-position function and preferably the anatomical model.

Turning back to Fig. 3, the method 300 comprises a step 310 of defining an estimated distance between each pair of field-sensitive sensors. Thus, in step 310, an estimated distance is defined or initiated for each pair of field-sensitive sensors. It will be apparent from the following description that the estimated distance(s) is/are to be modified to determine the inter-sensor distance(s).

In one example, step 310 comprises defining each estimated distance to be equal to a predetermined distance (e.g., 0 mm or 5 mm or other). The predetermined distance may be the same or different for each pair of field-sensitive sensors. They may depend on the design (e.g., shape) of the interventional device carrying the sensors.

In another example, step 310 comprises receiving an estimated distance for each pair of sensors, e.g., from a user input provided at a user interface. In this way, a user or subject is able to provide information on the estimated distance(s). This can allow the subject, for instance, to input manufacturer-provided information on the estimated distances or the like.

In yet another example, step 310 comprises receiving an estimated distance for each pair of sensors from a database, memory, or other storage unit. For instance, a database may store a dataset or data table that maps identifiers of an interventional device to different sets of one or more estimated distances. An identifier of an interventional device may be obtained, e.g., via a user input or scanning device, such as a bar-code scanner, and used to identify the estimated distance(s) for that interventional device using the dataset or data table stored by the database.

The estimated distances may be the same as the reference distances mentioned hereinbefore.

The method then performs a position prediction process 320 a plurality of times.

The position prediction process comprises a number of steps 321, 322, 323 that are performed for each of a plurality of different positions or locations of the sensors and/or interventional device within the crossing electromagnetic fields.

Each position may be a different position within an anatomical cavity of a subject. Thus, each of the plurality of different positions of the interventional device may be a different position within an anatomical cavity of a subject. In this way, the determination of the inter-sensor distances may take place in the same environment in which later mapping or locating-determining takes place.

Alternatively, although less preferred for reasons of convenience, each position may be a different position within a calibration chamber. The calibration chamber may, for instance, contain a fluid or the like. The fluid maybe fluid that resembles or is blood for improved accuracy.

In any event, each position is a different position in R-space within crossing electromagnetic fields.

The position prediction process 320 comprises a step 321 of receiving a set of measurements for each field-sensitive sensor. As previously explained, each measurement in a set of measurements is a measurement of the (specifically, a single) field-sensitive sensor to a different (single) one of the crossing electromagnetic fields.

Thus, step 321 comprises receiving measurement data 159 comprising for each of a plurality of different positions of the interventional device within crossing electromagnetic fields, a set of measurements for each field-sensitive sensor.

The position prediction process 320 further comprises a step 322 of predicting a position of each field-sensitive sensor in a spatial co-ordinate system by processing the set of measurements using a measurement-to-position function. Examples of measurement-to-position functions are established in the art. As previously mentioned, suitable measurement-to-position functions are disclosed by the European Patent Applications EP 0775466 A2, EP 3568068 A1 and EP 3607879 A1.

For the purposes of step 322, a default or initial measurement-to-position function may be used. Such a measurement-to-position function may, for instance, be specific to the interventional device, e.g., the type or model of interventional device. The measurement-to-position function may, for instance, be defined by a manufacturer of the interventional device as a default measurement-to-position function. Alternatively, it may be defined by a manufacturer of a processing system that performs method 200.

The position prediction process 320 then performs a step 323 of predicting or determining, for each of the one or more pairs of field-sensitive sensors, a function-derived distance between each pair of field-sensitive sensors.

This is performed by, for each pair of field-sensitive sensors, at least calculating a distance between the positions, in the spatial co-ordinate system, of the field-sensitive sensors in the pair of field-sensitive sensors. Thus, the positions of the sensors defined in step 322 are used to derive or determine a function-derived distance. Approaches for determining a distance between two positions in a spatial co-ordinate system are well known in the art, e.g., determining the Euclidean or Pythagorean distance using an L2 norm or the like.

Thus, the step of calculating a distance between the positions, in the spatial co-ordinate system, of the field-sensitive sensors in the pair of field-sensitive sensors may comprise calculating the L2 norm of a vector that originates at the position, in the spatial co-ordinate system, of one of the field-sensitive sensors of the pair of field-sensitive sensors and terminates at the position, in the spatial coordinate system of the other of the field-sensitive sensors of the pair of field-sensitive sensors.

However, other magnitude or distance determining mechanisms could be used instead of the L2 norm, such as the L1 norm (i.e., the Manhattan distance) or the Chebyshev distance.

The method 300 then performs a minimization process 330. The minimization process 330 is repeated until one or more predetermined criteria are met, a number of suitable examples of which are provided later in this disclosure.

In the context of the present disclosure, the term "minimization process" is used to reflect the intention, but not requirement, of said process to reduce or "minimize" the value of a cost function. The output of the minimization process need not be a global minimum, but may rather represent a local minimum or be an output when certain predetermined criteria have been met (e.g., the cost function been minimized beyond a certain threshold, the process has been performed a certain number of times or an override indicator has been provided).

The minimization process comprises a step 331 of determining a value of a cost function that processes the function-derived distances and the estimated distance between each pair of field-sensitive sensors. Various approaches for performing, and possible variations of, step 331 will be described later in this disclosure.

The minimization process then moves to a step 332 of modifying the estimated distance between each pair of field-sensitive sensors responsive to the determined value of the combined cost function. Approaches for modifying values input into a cost function responsive to the determined value of a cost function are established in the art, and are commonly labelled iterative methods. In particular optimization algorithms, such as gradient descent algorithms can be used.

It is not necessary or essential for the measurement-to-position function used to generate the function-derived distances to be modified in step 332 or in the minimization process 330. In particular, in some embodiments, no modifications are made to the measurement-to-position function during the iterative performances of the minimization process. This approach significantly increases a speed and simplicity of determining the inter-sensor distances. It is recognized that performing a minimization process using data obtained from a large of number of positions of the interventional device (e.g., >100 or >500), then the inaccuracy of the measurement-to-position function used in step 322 will be averaged out when calculating the inter-sensor distances. If the method 300 is repeatedly performed, e.g., after more measurement data 159 is available meaning that the intervention device has moved to more positions, then the accuracy of method 300 will be increased.

Nonetheless, an example in which the measurement-to-position function is changed during the minimization process 330 will be later described, as this may be advantageous in some embodiments for improved accuracy.

After iteratively performing the minimization process, i.e., only once the one or more predetermined criteria have been met, the method 300 comprises a step 340 of, for each of the one or more pairs of field-sensitive sensors, defining the estimated distance between said pair of field-sensitive sensors, as the distance between the pair of field-sensitive sensors.

Step 340 may comprise providing the defined estimated differences to a further processing device or inputting the defined estimated differences into a further processing procedure, e.g., a mapping generation procedure for generating a mapping function. Other uses, e.g., for providing user-perceptible outputs or the like, for the defined estimated differences are provided later in this disclosure.

Step 340 may comprise outputting, for each of the one or more pairs of field-sensitive sensors, the defined distance. This information may be output, for instance, to a further processing unit such as a further processing module of the mapping system (e.g., for determining the measurement-to-position function).

Thus, the minimization process 330 performs an iterative method to determine the value(s) for the estimated distance(s) between the one or more pairs of field-sensitive sensors of the interventional device.

Determining the combined cost function in step 332 may be performed by combining (e.g., summing, averaging, multiplying and/or a combination of the same) the values produced by performing a cost function for each pair of field-sensitive sensors at each of the plurality of different positions of the interventional device.

Thus, step 332 may comprise a sub-step 332A of determining, for each of the plurality of different positions of the interventional device, the value of a cost function for each sensor pair using a difference between the function-derived distance and the estimated distance and a sub-step 332B of determining a value of a combined cost function by combining the determined values of the cost function for each sensor pair.

As an example, one approach for determining a value of the combined cost function for step 332 may be performed using the following equation:

$$\sum_{i \in s} \sum_{k,l \in p} \left( \left| f(\mathbf{x}_k^i) - f(\mathbf{x}_l^i) \right| - \Delta_{k,l} \right)^2 \tag{1}$$

where i represents a position of the interventional device amongst a plurality s of positions, p represents a plurality of pairs of field-sensitive sensors, k represents one of the field-sensitive sensors in a pair of field-sensitive sensors and 1 represents the other of the field-sensitive sensors in the same pair of field-sensitive sensors. The function f(.) is the measurement-to-position function, the variable xk is the set of measurements for the field-sensitive sensor k at position i, the variable $\mathbf{x}_l^i$ is the set of measurements for the field-sensitive sensor 1 at position i. Thus, the output of function f(.) in equation (1) is a spatial-coordinate system position of field-sensitive sensor k or 1 at position i. The value $\Delta_{k,l}$ is the estimated distance between the pair of field-sensitive sensors k and 1.

The function |.| is preferably an L2 norm, i.e., the Euclidean or Pythagorean distance. However, other magnitude determining mechanisms could be used, such as the L1 norm (i.e., the Manhattan distance) or the Chebyshev distance.

It will be appreciated that the function of equation (2) therefore represents a cost function. The combined cost function of equation (1) represents the summing of the values of all cost functions for each sensor pair and each position of the interventional device.

$$\left( \left| f(\mathbf{x}_k^i) - f(\mathbf{x}_l^i) \right| - \Delta_{k,l} \right)^2 \tag{2}$$

Thus, determining the value of the combined cost function can be performed by determining, for each of the plurality of different positions of the interventional device, the value of a cost function (e.g., as defined in equation (2)) for each sensor pair using a difference between the function-derived distance and the estimated distance; and determining a value of a combined cost function by combining the determined values of the cost function for each sensor pair.

In particular, combining the determined values of the cost function may comprise summing the determined values of the cost function.

Determining a value of a cost function for each sensor pair may comprise, for each sensor pair, determining the square of the difference between the function derived distance and the estimated distance. An example of this approach is demonstrated by equation (2). In other words, the cost function of equation (2) is a squared error.

However, alternative forms of cost function could be used, e.g., a (non-squared) error.

Moreover, the proposed combined cost function (defined by equation (1)) effectively proposes to equally weight the deviations from estimated and function-derived distances for all pairs of sensors and all interventional device positions.

However, it has been recognizes that, due at least to manufacturing or production processes, the distance for some sensor pairs (particularly the two most distal field-sensitive sensors for a catheter) has a greater variability across a production batch or line than other sensor pairs.

An alternative cost function could therefore more heavily weight, in the combined cost function, cost functions for some sensor pairs than other sensor pairs, and in particular, could weight the cost function for sensor pairs based on a known production variability (e.g., standard deviation).

As an example, the cost function of equation (2), or any other similar cost function, could include an additional weighting (e.g., a scaling factor) that is responsive to a production variability (e.g., standard deviation) of the sensor pair k,l. The weight may, for instance, be e.g., inversely proportional to the standard deviation.

This approach effectively employs Bayes estimation theory to improve the estimation of the inter-sensor distance.

As another example, the cost function of equation (2), or any other similar cost function, could include an additional weight (e.g., a scaling factor) that is responsive to an expected or known noise of one or both of the pair of sensors k,l. The weight may, for instance, be inversely proportional to the expected noise (e.g., signal-to-noise ratio) for each or both sensors in the pair of sensors. The expected noise may be determined in a prior calibration process and/or from literature, such as those produce by the manufacturer of the interventional device.

In this way, the cost function(s) is/are more specifically tuned to the expected error distribution of a given catheter type.

To accelerate the speed and/or efficiency of the minimization process 330, the estimated distance $\Delta_{k,l}$ between each pair of sensors k, l can be conceptually split into a pre-calibration distance $d_{k,l}$ and a deviation distance $\delta_{k,l}$. The pre-calibration distance $d_{k,l}$ may be the distance obtained in step 310, and may remain fixed throughout the minimization process 330. The deviation distance $\delta_{k,l}$ may be the value modified during the minimization process 330, to thereby modified the estimated distance.

In this way, the combined cost function may be defined as follows:

$$\sum_{i \in s} \sum_{k,l \in p} \left( \left| f\left(\mathbf{x}_k^i\right) - f\left(\mathbf{x}_l^i\right) \right| - d_{k,l} + \delta_{k,l} \right)^2 \qquad (3)$$

and the minimization process may aim to modify $\delta_{k,l}$ (for all s and p) to reduce or minimize the value of the combined cost function (3).

In an ideal case, the value of $\delta_{k,l}$ (for all s and p) will equal zero, but in reality, this term allows for adaption of the inter-sensor(s) distance to the properties of the interventional device that is actually used.

In the above-described method 300, it has been explained how it is not essential (any may be preferred) to modify the measurement-to-position function f(.) during the minimization process 300. This approach is less computationally expensive, likely more robust and can be integrated more closely with existing code/approaches than approaches that modify both the measurement-to-position function and the estimated distance(s).

Fig. 3 illustrates an alternative minimization process 400 (which can replace minimization process 330 of Fig. 2), in which modifications are also made to the measurement-to-position function during the minimization process. This approach thereby simultaneously determines the inter-sensor distance and determines the measurement-to-position function.

The minimization process 400 again comprises a step 321 of determining a value of a combined cost function that processes the function derived distances and the estimated distance between each pair of field-sensitive sensors. The minimization process also comprises a step 322 of modifying the estimated distance between each pair of field-sensitive sensors responsive to the determined value of the combined cost function.

However, the minimization process further comprises a step 410 of modifying the measurement-to-position function responsive to the determined value of the combined cost function. Approaches for modifying the measurement-to-position function may employ any known approach established in the art, which provide a wide variety of modification techniques for modifying the measurement-to-position function responsive to a value of a cost function.

The method may then perform a step 420 of predicting a position of each field-sensitive sensor in a spatial co-ordinate system by processing the set of measurements using a measurement-to-position function. Step 420 is effectively a repeat of step 322 performed in method 300, but using the updated measurement-to-position function.

The method may then perform a step 430 of predicting, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors. Step 430 is effectively a repetition of step 323 performed in method 300, but using the function-derived distances derived from the updated measurement-to-position sensor.

The minimization process 400 can be represented by the following equation:

$$\arg\min_{\theta, \Delta_{k,l}} \sum_{i \in s} \sum_{k,l \in p} \left( \left| f\left(\mathbf{x}_k^i; \theta\right) - f\left(\mathbf{x}_l^i; \theta\right) \right| - \Delta_{k,l} \right)^2 , \qquad (4)$$

$$\text{s. t.} \sum \Delta_{k,l} = \sum d_{k,l} = \text{const},$$

where θ represents the parametrization of the measurement-to-position function, such that $f\left(\mathbf{x}_k^i; \theta\right)$ represents the spatial co-ordinate system position of field-sensitive sensor k, for position i of the interventional device, derived using the measurement-to-position function parametrized by θ.

In some examples, the minimization process 400 further comprise a constraint generation procedure 420. The constraint generation procedure is configured to generate additional constraints for the modification and/or minimization of the estimated distance(s) and/or the measurement-to-position function.

The constraint generation procedure 420 comprises a step 421 of generating an anatomical model using the determined positions for the field-sensitive sensors from the measurement data. Approaches for generating an anatomical model have been previously described in the context of a mapping system.

The constraint generation procedure 420 comprises a step 422 of comparing the anatomical model to secondary imaging data. The secondary imaging data may be any imaging data of the anatomical cavity generated in a separate imaging procedure, e.g., an X-ray imaging procedure, a CT imaging procedure, an MR imaging procedure and/or an ultrasound imaging procedure. The secondary imaging data may be obtained before the interventional device is inserted into the subject/subject.

The constraint generation procedure 420 also comprises a step 423 of generating one or more constraints for the minimization process, e.g., the steps of modifying the estimated distance(s) and/or the measurement-to-position function.

Step 422 may, for instance, comprise a sub-step of determining one or more model-derived inter-landmark distances and a sub-step of determining one or more corresponding image-derived inter-landmark distances.

Each model-derived inter-landmark distances is a predicted distance between two landmarks of an anatomical structure (represented in the anatomical model) that has been generated by processing the anatomical model.

Each image-derived inter-landmark distance, which corresponds to a particular model-derived inter-landmark distance, is a predicted distance between the same two landmarks of the anatomical structure as for the corresponding model-derived inter-landmark distance, that has been generated by processing the secondary imaging data. Approaches for determining an inter-landmark distance from imaging data are well-established in the art.

A landmark is an identifiable feature or element of an anatomical structure, e.g., an entrance or exit to a particular blood vessel or a particular identifiable anatomical point.

Step 422 may then comprise determining a difference between each model-derived inter-landmark distance and its corresponding image-derived inter-landmark distance. This difference can be labelled an inter-landmark difference.

Step 423 may comprise setting one or more constraints using the determined differences. In particular, the one or more constraint may be to restrict a maximum allowable difference between the differences derived in step 422.

The method 300 determines or estimates the distance between one or more pairs of field-sensitive sensors carried by an interventional device. It is not essential that the one or more pairs includes all possible pairs of the field-sensitive sensors.

Preferably, the one or more pairs contains only those pairs that are used in determining the measurement-to-position function for the interventional device, which can vary depending upon the type or model of the interventional device. By way of example, some measurement-to-position functions only make use of distances between pairs of sensors that are adjacent to one another. Thus, the one or more pairs may comprise all pairs of adjacent sensors.

Thus, the one or more pairs of field-sensitive sensors may comprise only a subset of all possible pairs of field-sensitive sensors of the interventional device, the subset comprising a non-integer fraction or portion of the possible pairs of the field-sensitive sensors.

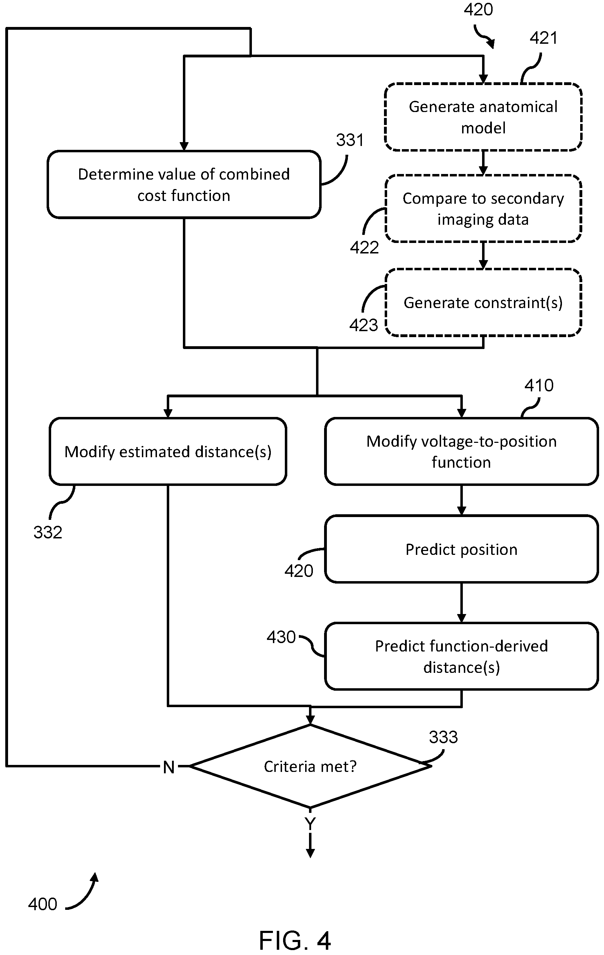

The minimization function 330 (described with reference to Fig. 3) and minimization function 400 (described with reference to Fig. 4) is performed until one or more predetermined criteria are met. Thus, the minimization function 330, 400 may comprise a step 333 of determining whether or not the one or more predetermined criteria have been met. In responsive to a positive determination in step 333, the minimization function 330 ends, otherwise the minimization function is repeated.

Although illustrated as occurring after other steps of the minimization function 300, 400, step 333 may instead take place at any other time during the minimization function (e.g., at the start of the minimization function or after the value of a combined cost function has been determined).

The predetermined criteria may be otherwise labelled a stoppage or termination criteria. Stoppage or termination criteria are reasonably well known in the field of minimization processes, and the following examples are provided merely for the sake of full understanding and explanation.

A first predetermined criterion may be that the minimization process has been iteratively performed no less than a first predetermined number of iterations. The first predetermined number may be a number larger than 100, e.g., larger than 1000.

A second predetermined criterion may be the value of the combined cost function changing by less than a first predetermined percentage or amount over a preceding second predetermined number of iterations. The first predetermined percentage may, for instance, be less than 3%, e.g., less than 1%, e.g., less than 0.5%.

If the second predetermined criterion is used, the combined cost function may be recalculated (e.g., step 331 may be repeated) after the estimated distance(s) (and, if modified, the function-derived distances) have been determined. Thus, step 333 may comprise repeated step 331 before determining whether the second predetermined criterion has been met.

A third predetermined criterion may be the value of a second cost function differing by less than a predetermined amount, wherein the value of the second cost function is responsive to one or more inter-landmark differences. If the third predetermined criteria is used, steps 421 and 422 (previously described) could be performed or repeated to determine or derive the inter-landmark differences. A simple example of a second cost function is a summing of the inter-landmark difference(s) and/or a summing of the squares of the inter-landmark difference(s).

A fourth predetermined criterion may be that of an override indicator being provided. The override indicator may be provided, for instance, by an operator of the interventional device at a user input interface and/or by a separate system (e.g., the mapping system) responsive to some predetermined logic or instructions. Thus, for instance, the override indicator may act as an interrupt or other similar computational interruption mechanism.

Other predetermined criteria may contain a combination of any of the above-identified criteria.

The method 300 determines or estimates the distance between one or more pairs of field-sensitive sensors using sets of measurements obtained at a plurality of positions of the interventional device within crossing electromagnetic fields, i.e., measurement data comprising (for a plurality of positions) sets of measurements.

In some examples, instead of using the sets of measurements from all possible positions of the interventional device, more accurate determination of the inter-sensor distance can be made by determining the inter-sensor distance using the sets of measurements from only a subset (i.e., not all) of the positions of the interventional device.

It is recognized that the positions of the interventional device may include positions within the blood pool of the subject (where the crossing electromagnetic fields are more linear) as well as samples touching the tissue or bounds of the anatomical cavity (where the crossing electromagnetic fields change non-linearly).

In some examples, to improve the accuracy of the estimation of the inter-sensor distances, the measurement data used by method 300 may only include sets of measurements for positions where the interventional device does not touch the bounds of the anatomical cavity, i.e. an anatomical structure.

Approaches for determining whether the interventional device is touching the bounds of an anatomical cavity are established in the art, e.g., as set out in European Patent Application having publication number EP 3,932,351 A1 or International Patent Application having publication number WO 2019/215721 A1.

It is also recognized that the application of ablation (e.g., and associated flushing) may distort the measurements of the field-sensitive sensors. These can be excluded from the measurement data used to estimate the inter-sensor distances.

Thus, in examples in which the interventional device is an ablation interventional device, to improve the accuracy of the estimation of the inter-sensor distances, the measurement data used by method 300 may only include sets of measurements obtained or sampled at positions when no ablation is performed by the interventional device. Determination of whether or not ablation is being performed can be established by communication with an ablation controller or processor.

In some examples, outlier detection of the measurement data can take place, with outlying sets of measurements being identified and discarded (along with the other sets for the corresponding positions) from the measurement data

The method 300 may be repeated two or more times, e.g., during a single interventional process. Each time the method 300 is repeated, new sets of measurements may be received in step 321 that have been sampled or otherwise obtained by the interventional device in the period since the last repetition of method 300.

The cost function generated in step 331 may make use of function-derived distances generated from the new sets of measurements and existing or previously generated function-derived distances. If the minimization process modifies the measurement-to-position function, then step 322 may comprise determining a position for each new set and each previously received set (i.e., sets received and processed in previous repetitions of method 300).

For instance, method 300 may be repeated every X minutes, where X is a real positive number such as 5 or 10. As another example, method 300 may be repeated every time more than Y additional sets of measurements become available for processing, where Y is a real positive number preferably larger than 500, e.g., 1000 or 5000.

Repeating the method 300 can minimize or reduce the effect of transient changes due to, for instance, biochemical effects in blood and/or caused by ablation or other surgical procedures within the subject.

If method 300 is repeated, the change of the (effective) sensor distances over time may be monitored to ensure localization quality. Sudden, large changes, due to e.g., charring, biochemical changes, and others, could be signaled to an operator, e.g., using an output interface.

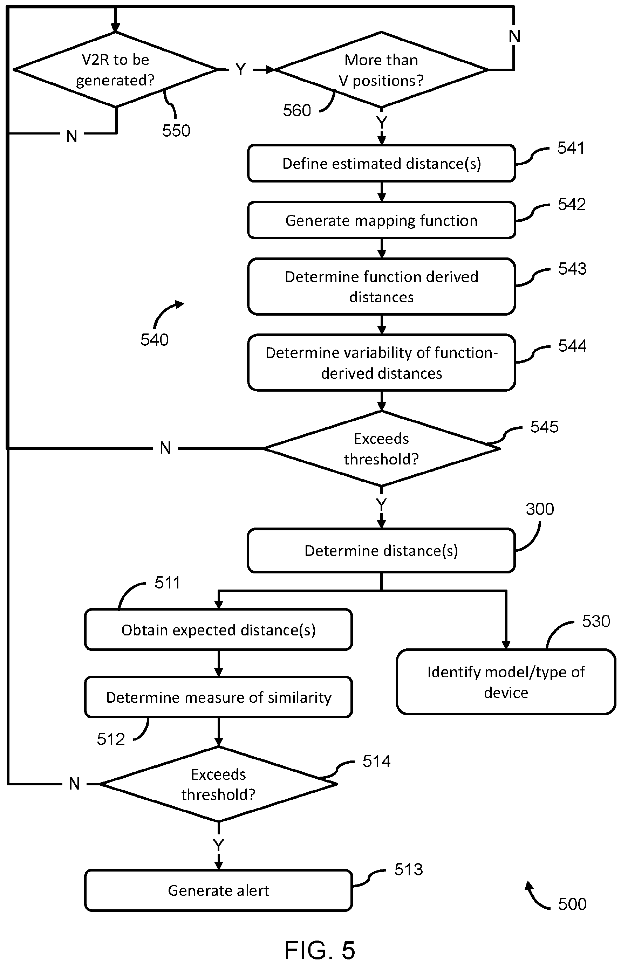

Fig. 5 illustrates a method 500 providing some additional optional steps or processes of a computer-implemented method incorporating the computer-implemented 300 previously described. In particular, Fig. 5 illustrates some optional proceeding steps (e.g., steps that may trigger the performance of computer-implemented method 300) or following steps (e.g., steps that may follow the performance of method 300).

In one example, the method 500 comprises a step 511 of obtaining, for each pair of field-sensitive sensors, an expected distance between said pair of field-sensitive sensors. The expected distance may, for instance, be an expected distance for the type or model of interventional catheter. This may be provided by an operator of the interventional catheter or, for instance, extracted from a database of expected distances. Approaches used for defining the estimated distance in step 310 of method 300 may be equally implemented for performing step 511.

The method 500 may also comprise a step 512 of determining, for all of the one or more pairs of field-sensitive sensors a measure of similarity between the expected distance and the defined distance. The measure of similarity may, for example, be the average of the differences between each defined distance and corresponding expected distance.

The method 500 may also comprise a step 513 of generating an alert responsive to the measure of similarity exceeding a predetermined threshold, e.g., as determined in a determination step 514. This approach provides a technique for alerting an operator if the interventional device does not match an expected interventional device. This can, for instance, alert an operator to a scenario in which they are using an incorrect device, or where the characteristics of the used device do not match the expected characteristics.

Generating an alert may comprise, for instance providing a user-perceptible output (e.g., a visual output or an audible output) at an output interface.

The method 500 may comprise a step 530 using the defined estimated distance(s) to identify the type and/or model of the interventional device. In particular, a device-identifying dataset (e.g., stored by a database or other memory unit) may store identifiers of types/models of interventional device and the expected inter-sensor distances. The inter-sensor distances stored by this dataset that most closely match the defined estimated distance(s) may be identified, and the corresponding identifier of the type/model of interventional device retrieved. This information can, for instance, be provided to an operator of the interventional device using an output interface, e.g., in the form of a visual representation of the identified type/model of the interventional device and/or an audio representation of the identified type/model of the interventional device.

In some examples, the method 500 comprises a calibration trigger process 540. The calibration trigger process is configured to determine whether or not a calibration process, e.g., the method 300, is to be performed.

In one example, the calibration trigger process 530 comprises a step 541 of defining an estimated distance between each pair of field-sensitive sensors. This may be performed using any process described with reference to step 310.

If the calibration trigger process is performed when seeking to repeat the method 300 (e.g., as later described), then the estimated distances defined in step 541 may be the distances defined by a previous iteration of method 300.

The calibration trigger process 540 may then determine in step 542 a mapping function using the estimated distance(s). This can be performed by obtaining a plurality of sets of measurements and determining a mapping function using the sets of measurements and the estimated distance(s), e.g., using the estimated distance(s) as a constraint or part of a cost function a minimization process performed on the parameters of mapping function

The calibration trigger process 540 may then perform a step 543 of determining function derived distances

The calibration trigger process 540 may then perform a step 544 of determining a variability, e.g., standard deviation, of function-derived distances between field-sensitive sensors for a plurality of positions of the interventional device. Here, a function-derived distance is again a distance between a pair of field-sensitive sensors determined from the positions of the field-sensitive sensors in the spatial co-ordinate system.

The calibration trigger process 540 may then perform a determining step 545 of determining whether or not the variability exceeds a predetermined variability threshold. This will indicate whether or not the variability of the function-derived distances is too large (indicating that the mapping function and/or the estimated distances used to constrain the mapping function are inaccurate.

In response to a positive determination in step 545, the method 300 may be performed. In response to a negative determination in step 545, the method 300 does not need to be performed (i.e., may not be performed) and the estimated distances can be used as the determined distances between the pair(s) of field-sensitive sensors.

During the course of conventional or existing procedures that make use of a measurement-to-position function, it is relatively common for the measurement-to-position function to be updated or re-determined (e.g., as more sets of measurements become available).

In some examples, the method 300 may be repeated each time a new measurement-to-position function is determined. Thus, the method 500 may comprise a step 550 of determining whether a new measurement-to-position function is to be determined.

In other examples, the method 300 may be repeated for only a predetermined number of times the measurement-to-position function is calculated and/or updated, e.g., for only the first 10 times the measurement-to-position function is updated.

In some examples, once the inter-sensor distance(s) have been determined with an acceptable level of accuracy, then performance of method 300 does not need to be continued for future updates of the measurement-to-position function.

The method 500 may comprise a step 560 of determining for how many positions of the interventional device are there corresponding sets of measurements available. The method 300 may, for instance, only be performed if sets of measurements are available for more than a predetermined number (V) of positions of the interventional device within the crossing electromagnetic fields. The predetermined number of positions may be no less than 50, e.g., no less than 100, e.g., no less than 500.

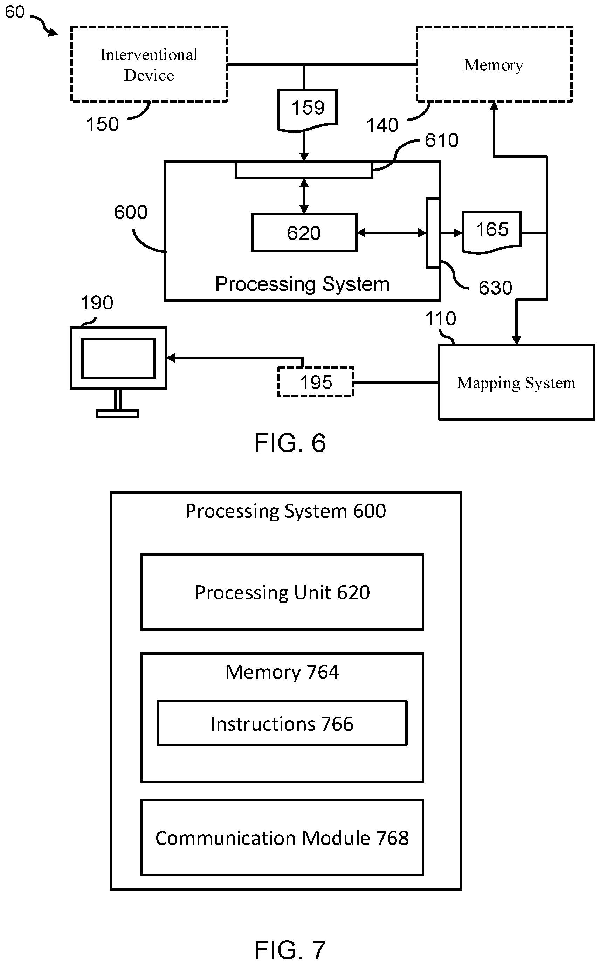

Fig. 6 illustrates a processing system 600 for determining, for each of one or more pairs of field-sensitive sensors, a distance between said pair of field-sensitive sensors, wherein each field-sensitive sensor is carried by an interventional device. The processing system may form part of the mapping system illustrated in Fig. 1, or may be a separate system configured to receive data generated by the mapping system.

In particular, the processing system 600 may form part of a larger system 60, which also provides an embodiment.

The processing system 600 comprises an input interface 610 configured to receive, for each of a plurality of different positions of the interventional device within crossing electromagnetic fields, a set of measurements for each field-sensitive sensor, each measurement in the set of measurements being a measurement of the field-sensitive sensor to a different one of the crossing electromagnetic fields.

Thus, the input interface 610 may receive measurement data 159 comprising for each of a plurality of different positions of the interventional device within crossing electromagnetic fields, a set of measurements for each field-sensitive sensor.

The measurement data 159 may be obtained from the interventional device or the mapping system. Alternatively, the measurement data 159 may be obtained from a memory 140. When the processing system 600 is integrated into the mapping system, the measurement data 159 may be obtained from another module of the mapping system and/or a memory or storage unit.

The processing system 600 also comprises a processing unit 620 communicatively coupled to the input interface.

The processing unit 620 is configured to determine the distance between each of the one or more pairs of field-sensitive sensors, using at least the obtained measurement data.

The processing system may be appropriately adapted to perform any herein described method, as would be readily apparent to the skilled person.

The processing system may further comprise an output interface 630 configured to output, for each of the one or more pairs of field-sensitive sensors, the defined distance. This information may be output, for instance, to a further processing unit such as a further processing module or the mapping system (e.g., for determining the measurement-to-position function). In some examples, the information may be provided to a/the memory 140 or other storage unit.

The system 60 may comprise a user interface 190 or output interface. The user/output interface 190 may be configured to provide a visual representation of the anatomical model. In some examples, a display processor 195 may be configured to receive the anatomical model from the mapping system 110 and render the anatomical model to generate display data that can be received and processed by the user/output interface 190 for providing a visual representation of the anatomical model.

Fig. 7 is a schematic diagram of a processing system 600, according to embodiments of the present disclosure. The processing system 600 is configured to carry out a method according to an embodiment, such as the method 300 described with reference to Fig. 3.

As shown, the processing system 600 may include a (data) processing unit 620, a memory 764, and a communication module 768. These elements may be in direct or indirect communication with each other, for example via one or more buses.

The processing unit 620 may include a central processing unit (CPU), a digital signal processor (DSP), an ASIC, a controller, an FPGA, another hardware device, a firmware device, or any combination thereof configured to perform the operations described herein. The processing unit 620 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g., formed of a set of distributed processors.

The memory 764 may include a cache memory (e.g., a cache memory of the processing unit 620), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 764 includes a non-transitory computer-readable medium. The non-transitory computer-readable medium may store instructions. For example, the memory 764, or non-transitory computer-readable medium may have program code recorded thereon, the program code including instructions for causing the processing system 600, or one or more components of the processing system 600, particularly the processing unit 620, to perform the operations described herein. For example, the processing system 600 can execute operations of the method 500.

Instructions 766 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 764, with the code recorded thereon, may be referred to as a computer program product.

The communication module 768 can include any electronic circuitry and/or logic circuitry to facilitate direct or indirect communication of data between the processing system 600, the penetration device and/or the user interface (or other further device). In that regard, the communication module 768 can be an input/output (I/O) device. In some instances, the communication module 768 facilitates direct or indirect communication between various elements of the processing system 600.

In the context of the present disclosure, the distance between two field-sensitive sensors or the inter-sensor distance may refer to an "effective distance" between a pair of field-sensitive sensors. The effective distance represents the electrical distance between the two points at which the electromagnetic fields are being sampled. This is distinguishable from the geometrical distance, being a distance between the geometric center of each sensor.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figs. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

It will be appreciated that different disclosed processing systems may form part of a single processing system, e.g., each disclosed processing system is a sub-system of a single processing system. In the context of the present disclosure, this means that a single system could perform the actions of a mapping system and/or a processing system and/or a field controller processing system as herein described.

An input/output interface may represent internal circuitry of a larger system, e.g., connected to an input/output of a processing unit. The same processing unit may be reused to perform different tasks, such as: generating the anatomical model and modifying the anatomical model (or 3D mesh).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

**Claims**

1. A computer-implemented method of determining, for one or more pairs of field-sensitive sensors carried by an interventional device, a distance between said pair of field-sensitive sensors, the computer-implemented method comprising:

   defining an estimated distance between each pair of field-sensitive sensors;
   for each of a plurality of different positions of the interventional device within crossing electromagnetic fields:

      receiving, a set of measurements for each field-sensitive sensor, each measurement in the set of measurements being a measurement of the field-sensitive sensor to a different one of the crossing electromagnetic fields;
      predicting a position of each field-sensitive sensor in a spatial co-ordinate system by processing the set of measurements using a measurement-to-position function;
      predicting, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors;

   iteratively performing a minimization process until one or more predetermined criteria are met, the minimization process comprising:

      determining a value of a combined cost function that processes the function derived distances and the estimated distance between each pair of field-sensitive sensors; and
      modifying the estimated distance between each pair of field-sensitive sensors responsive to the determined value of the combined cost function;

   after iteratively performing the minimization process, for each of the one or more pairs of field-sensitive sensors, defining the estimated distance between said pair of field-sensitive sensors, as the distance between the pair of field-sensitive sensors.

2. The computer-implemented method of claim 1, wherein no modifications are made to the measurement-to-position function during the iterative performances of the minimization process.

3. The computer-implemented method of claim 1, wherein the minimization process further comprises:

   modifying the measurement-to-position function responsive to the determined value of the combined cost function;
   repeating the step of predicting a position of each field-sensitive sensor in a spatial coordinate system by processing the set of measurements using a measurement-to-position function; and
   repeating the step of predicting, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors.

4. The computer-implemented method of any of claims 1 to 3, wherein the step of determining the value of the combined cost function comprises:

   determining, for each of the plurality of different positions of the interventional device, the value of a cost function for each sensor pair using a difference between the function-derived distance and the estimated distance; and
   determining a value of a combined cost function by combining the determined values of the cost function for each sensor pair.

5. The computer-implemented method of claim 4, wherein combining the determined values of the cost function comprise summing the determined values of the cost function.

6. The computer-implemented method of claim 4 or 5, wherein determining a value of a cost function for each sensor pair comprises, for each sensor pair, determining the square of the difference between the function derived distance and the estimated distance.

7. The computer-implemented method of any of claims 1 to 6, wherein the one or more pairs of field-sensitive sensors comprises only a subset of all possible pairs of field-sensitive sensors of the interventional device, the subset comprising a non-integer fraction of the possible pairs of the field-sensitive sensors.

8. The computer-implemented method of any of claims 1 to 7, wherein the step of calculating a distance between the positions, in the spatial co-ordinate system, of the field-sensitive sensors in the pair of field-sensitive sensors comprising calculating the L2 norm of a vector that originates at the position, in the spatial co-ordinate system, of one of the field-sensitive sensors of the pair of field-sensitive sensors and terminates at the position, in the spatial co-ordinate system of the other of the field-sensitive sensors of the pair of field-sensitive sensors.

9. The computer-implemented method of any of claims 1 to 8, wherein the plurality of different positions of the interventional device comprises no fewer than 100 different positions, and more preferably, no fewer than 500 different positions.

10. The computer-implemented method of any of claims 1 to 9, wherein the one or more predetermined criteria comprise:

   the minimization process being iteratively performed no less than a first predetermined number of iterations;
   the value of the combined cost function changing by less than a first predetermined percentage or amount over a preceding second predetermined number of iterations; and/or
   an override indicator being provided.

11. The computer-implemented method of any of claims 1 to 10, further comprising:

   obtaining, for each pair of field-sensitive sensors, an expected distance between said pair of field-sensitive sensors;
   determining, for all of the one or more pairs of field-sensitive sensors a measure of similarity between the expected distance and the defined distance; and
   generating an alert responsive to the measure of similarity exceeding a predetermined threshold.

12. The computer-implemented method of any of claims 1 to 11, wherein each of the plurality of different positions of the interventional device is a different position within an anatomical cavity of a subject.

13. A computer program product comprising computer program code means which, when executed on a computing device having a processing system, cause the processing system to perform all of the steps of the method according to any of claims 1 to 12.

14. A processing system for determining, for each of one or more pairs of field-sensitive sensors carried by an interventional device, a distance between said pair of field-sensitive sensors, the processing system comprising:

   an input interface configured to receive, for each of a plurality of different positions of the interventional device within crossing electromagnetic fields, a set of measurements for each field-sensitive sensor, each measurement in the set of measurements being a measurement of the field-sensitive sensor to a different one of the crossing electromagnetic fields;
   a processing unit configured to:

   define an estimated distance between each pair of field-sensitive sensors;
   for each of a plurality of different positions of the interventional device within crossing electromagnetic fields:

   predict a position of each field-sensitive sensor in a spatial co-ordinate system by processing the set of measurements using a measurement-to-position function;
   predict, for each of the one or more pairs of field-sensitive sensors, a function-derived distance between each pair of field-sensitive sensors by at least calculating a distance between the positions, in the spatial co-ordinate system, of the field-sensitive sensors in the pair of field-sensitive sensors;

   iteratively perform a minimization process until one or more predetermined criteria are met, the minimization process comprising:

   determining a value of a combined cost function that processes the function derived distances and the estimated distance between each pair of field-sensitive sensors; and

modifying the estimated distance between each pair of field-sensitive sensors responsive to the determined value of the combined cost function;

after iteratively performing the minimization process, for each of the one or more pairs of field-sensitive sensors, define the estimated distance between said pair of field-sensitive sensors, as the distance between the pair of field-sensitive sensors.

15. The processing system of claim 14, further comprising an output interface configured to output, for each of the one or more pairs of field-sensitive sensors, the defined distance.

FIG. 1

FIG. 2

159

Define estimated distance(s)

310

Receive voltage response data

321

Determine position for each set

322

320

Determine function-derived distance(s)

323

Determine values of cost function

331

330

Combine values

332

332A

Modify estimated distance(s)

332B

N — Criteria met?

333

Y

Define determined distance(s)

340

300

FIG. 3

FIG. 4

FIG. 5

60

Interventional
Device

150

159

Memory

140

610

600

620

165

630

Processing System

190

110

195

Mapping System

FIG. 6

Processing System 600

Processing Unit 620

Memory 764

Instructions 766

Communication Module 768

FIG. 7

Europäisches
Patentamt

European
Patent Office

Office européen
des brevets

# EUROPEAN SEARCH REPORT

Application Number

EP 22 18 4948

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/221643 A1 (OLSON ERIC S [US]) 11 September 2008 (2008-09-11) * abstract; claim 1 * * paragraphs [0042] – [0045] * * the whole document * | 1-15 | INV. A61B5/287 A61B5/367 A61B5/06 |
| A | US 2020/289025 A1 (DICHTERMAN ELI [IL] ET AL) 17 September 2020 (2020-09-17) * paragraphs [0453] – [0454] * | 1-15 | ADD. A61B34/20 |

TECHNICAL FIELDS
SEARCHED      (IPC)

A61B

The present search report has been drawn up for all claims

| Place of search | Date of completion of the search | Examiner |
|---|---|---|
| The Hague | 13 January 2023 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 4948

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | | Publication date | Patent family member(s) | | Publication date |
|---|---|---|---|---|---|
| US 2008221643 | A1 | 11-09-2008 | CN | 101657153 A | 24-02-2010 |
| | | | EP | 2120700 A1 | 25-11-2009 |
| | | | JP | 5419717 B2 | 19-02-2014 |
| | | | JP | 2010520780 A | 17-06-2010 |
| | | | US | 2008221438 A1 | 11-09-2008 |
| | | | US | 2008221643 A1 | 11-09-2008 |
| | | | US | 2017172454 A1 | 22-06-2017 |
| | | | WO | 2008112039 A1 | 18-09-2008 |
| US 2020289025 | A1 | 17-09-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3568068 A1
- EP 0775466 A2
- EP 3607879 A1
- EP 3932351 A1
- WO 2019215721 A1

### Non-patent literature cited in the description

- **BERGER, MATTHEW et al.** A survey of surface reconstruction from point clouds. *Computer Graphics Forum.*, 2017, vol. 26 (1